# EUROPÄISCHE PATENTSCHRIFT

(11) **EP 3 405 097 B1**
(45) Veröffentlichungstag und Bekanntmachung des Hinweises auf die Patenterteilung: **04.03.2020**
(21) Anmeldenummer: 17700423.1
(22) Anmeldetag: 13.01.2017
(51) Int. Cl.: A61B 5/00, G01B 11/24, G01B 11/25

(54) **VORRICHTUNG ZUM DREIDIMENSIONALEN ERFASSEN EINER OBERFLÄCHENSTRUKTUR**
DEVICE FOR THE THREE-DIMENSIONAL CAPTURE OF A SURFACE STRUCTURE
DISPOSITIF DE DÉTECTION TRIDIMENSIONNELLE D'UNE STRUCTURE DE SURFACE

(30) Priorität: 19.01.2016 DE 102016000415; 19.01.2016 DE 102016000416
(43) Veröffentlichungstag der Anmeldung: 28.11.2018
(73) Patentinhaber: Dürr Dental SE, 74321 Bietigheim-Bissingen (DE)
(72) Erfinder: BAUER, Walter, 71735 Eberdingen-Nussdorf (DE)
(74) Vertreter: Ostertag & Partner Patentanwälte mbB
(86) Internationale Anmeldenummer: PCT/EP2017/050732
(87) Internationale Veröffentlichungsnummer: WO 2017/125328

(56) Entgegenhaltungen:
- WO-A2-2015/177784
- DE-A1-102012 111 008
- DE-A1-102014 002 514

## Beschreibung

Die vorliegende Anmeldung nimmt die Prioritäten der älteren Anmeldungen DE 10 2016 000 415.0 vom 19.01.2016 und DE 10 2016 000 416.9 vom 19.01.2016 in Anspruch.

### HINTERGRUND DER ERFINDUNG

### 1. Gebiet der Erfindung

Die Erfindung betrifft eine Vorrichtung zum dreidimensionalen Erfassen einer Oberflächenstruktur, insbesondere zum Erfassen von Zähnen,
a) mit einer chromatisch-konfokalen Optik, die eine optische Achse umfasst, und
b) mit einer Lichtquelle, die ein Raster von lateral zur optischen Achse beabstandeten Einzellichtquellen aufweist,
c) wobei die Optik die Einzellichtquellen derart auf die Oberflächenstruktur abbildet, dass dort ein Raster von Messpunkten erzeugt wird, die voneinander lateral beabstandet sind,
d) wobei eine Bildaufnahmeeinheit optisch äquivalent zu den Einzellichtquellen angeordnet ist, um das Raster von Messpunkten zu erfassen.

### 2. Beschreibung des Standes der Technik

Aus der DE 10 2006 007 172 B4 ist eine Vorrichtungen bekannt, bei der eine chromatisch konfokale Messanordnung verwendet wird, um eine Oberflächenstruktur dreidimensional zu erfassen. Aus WO 2015/177784 A2 ist eine chromatisch konfokale Messanordnung bekannt, welche auf einer durchstimmbaren Lichtquelle beruht. Aus DE 102014002514 A1 ist eine chromatisch konfokale Messanordnung bekannt, welche auf einer breitbandigen Lichtquelle beruht, welche durch einen räumlichen Lichtmodulator spektral moduliert wird.

Der chromatisch-konfokalen Messanordnung liegt die Erkenntnis zugrunde, dass die Brechkraft einer Linse im Allgemeinen von der Wellenlänge des hindurchtretenden Lichts abhängig ist. Dadurch weist eine Linse je nach Wellenlänge räumlich unterschiedlich verlaufende Fokusflächen und damit auch Bildflächen auf. Dieser Effekt ist bei optischen Systemen als chromatische Aberration bekannt und wird als unerwünschter Bildfehler angesehen, der beispielsweise durch die Verwendung von Achromaten oder Apochromaten möglichst korrigiert werden soll.

Bei der chromatischen-konfokalen Messanordnung macht man sich jedoch die sogenannte chromatische Längsaberration (CLA) zu Nutze, indem Weißlicht über eine Optik mit einer möglichst großen CLA auf eine Oberfläche abgebildet wird. Auf diese Weise wird der kurzwellige, blaue Anteil des Lichts näher bei der Optik gebündelt als der langwellige, rote Anteil.

Das von der Oberfläche rückgestreute Licht wird über die chromatische Optik und einen Strahlteiler konfokal auf ein Spektrometer abgebildet.

Befindet sich die Oberfläche nun beispielsweise an jenem Ort, an welchem das blaue Licht gebündelt wird, gelangt das rückgestreute blaue Licht nahezu vollständig wieder in die Optik. Für das blaue Licht ist somit die chromatisch-konfokale Bedingung erfüllt. Das rote Licht hingegen wird aufgrund der unscharfen Fokussierung auf der Oberfläche größtenteils in Richtungen gestreut, die nicht in den konfokalen Strahlengang der Optik fallen. Insgesamt wird dadurch erheblich weniger rotes Licht auf das Spektrometer fallen.

Durch Bestimmen der spektralen Anteile des rückgestreuten Lichts mit Hilfe des Spektrometers kann daher die Entfernung der Oberfläche zur Messanordnung erfassen werden.

Um ein dreidimensionales Bild der Oberfläche zu erhalten, werden die bekannten chromatisch-konfokalen Messanordnungen über die zu erfassende Oberfläche gescannt.

Zur Vermeidung der Scanbewegung wird bei der Vorrichtung nach der DE 10 2006 007 172 B4 mit Hilfe einer Rasteranordnung von Einzellichtquellen auf der Oberfläche ein Raster von lateral beabstandeten Messpunkten erzeugt, deren Farbspektrum mit Hilfe eines hochauflösenden Bildwandlers mit Abbildungsoptik und eines vorgeschalteten Prismas oder Gitters bestimmt wird. Auf diese Weise erhält man die Topografie der Oberfläche, ohne dass die Messanordnung bezüglich des Objekts verschoben werden müsste. Nachteilig an den bisher verwendeten Vorrichtungen ist der relativ hohe aparative Aufwand und die Baugröße für die Kombination aus Prisma bzw. Gitter und Bildwandler mit Abbildungsoptik als Spektrometer.

### ZUSAMMENFASSUNG DER ERFINDUNG

Aufgabe der Erfindung ist es daher, eine Vorrichtung zum dreidimensionalen Erfassen einer Oberflächenstruktur, insbesondere zum Erfassen von Zähnen, anzugeben, bei der die Bildaufnahmeeinheit besser, insbesondere kleiner und kostengünstiger, realisiert werden kann.

Erfindungsgemäß wird die oben genannte Aufgabe mit einer Vorrichtung nach Anspruch 1 gelöst.

Der Erfinder hat erkannt, dass keine aufwendige Kombination aus Prisma bzw. Gitter und Bildwandler mit Abbildungsoptik als Spektrometer notwendig ist, wenn die unterschiedlich farbigen spektralen Anteile anstatt zeitgleich in zeitlicher Abfolge an der Bildaufnahmeeinheit auftreffen. Dadurch ersetzt die Zeitachse eine für das Erfassen der spektralen Anteile üblicherweise notwendige Dispersionsrichtung.

Beim Erfassen der von den Messpunkten stammenden Intensitäten in der Bildaufnahmeeinheit wird dann die zeitliche Abfolge der Beleuchtung berücksichtigt, um zu bestimmen, für welche Farbe die konfokale Bedingung am besten erfüllt ist. Daraus lässt sich dann für jeden Messpunkt der Abstand zur Oberflächenstruktur ermitteln. Entscheidend ist dabei jeweils die Abfolge innerhalb eines Paares von Einzellichtquelle und zugehörigem Messpunkt. Untereinander kann die zeitliche Abfolge der spektralen Zusammensetzung an den Einzellichtquellen unterschiedlich sein.

Unter unterschiedlicher spektraler Zusammensetzung des von den Einzellichtquellen in zeitlicher Abfolge abgegebenen Lichts ist hier vor allem eine unterschiedliche Farbe des Lichts insbesondere mit einem jeweils möglichst engen Wellenlängenband zu verstehen. Vorzugsweise ist die zeitliche Abfolge der spektralen Zusammensetzungen so gewählt, dass das Zentrum des Wellenlängenbandes sich kontinuierlich verändert. Obwohl je nach gewünschter Auflösung auch voneinander beabstandete Wellenlängenbänder denkbar sind, folgen die Wellenlängenbänder vorteilhaft jedoch spektral dicht aufeinander.

Als Lichtquelle, die eine solche zeitliche Abfolge der Beleuchtung erzeugen kann, kommen beispielsweise unterschiedlich farbige LEDs oder dergleichen in Frage, die auf denselben Messpunkt abgebildet werden. Vorzugsweise weist die Lichtquelle jedoch vor einem Rasterelement zum Erzeugen der Einzellichtquellen ein dispersives Element mit einer Dispersionsrichtung, insbesondere ein optisches Gitter mit einer Beugungsrichtung, und mindestens eine zumindest teilweise in Dispersionsrichtung ausgerichtete Zeile von einzeln ansteuerbaren breitbandigen Lichtquellen, vorzugsweise Weißlichtquellen, insbesondere LEDs, auf. Unter breitbandigen Lichtquellen wird hier vor allem verstanden, dass der Wechsel der Lichtquellen ohne die Verwendung des dispersiven Elements nicht zu einer ausreichenden Auflösung der chromatisch-konfokalen Bedingung führen würde. Durch die Verwendung des dispersiven Elements fällt beim Schalten der breitbandigen Lichtquellen dennoch nacheinander Licht mit unterschiedlicher Wellenlänge auf das Rasterelement, wodurch die Einzellichtquellen wiederum in entsprechender zeitlicher Abfolge ausreichend unterschiedlich farbiges Licht abgeben.

Als Rasterelement kann die Lichtquelle beispielsweise eine Lochrasterblende mit einem Raster von Blendenöffnungen umfassen. Dabei kann der Lochrasterblende ein Mikrolinsenarray vorgeschaltet sein. In diesem Zuge kann auch eine Kondensorlinse zur Anwendung kommen, um möglichst viel Lichtintensität in die Blendenöffnungen zu fokussieren. Alternativ können die Einzellichtquellen auch durch ein aufgespaltetes Lichtfaserbündel dargestellt werden.

Vorzugsweise ist die Steuer- und Auswerteeinrichtung derart eingerichtet ist, dass die breitbandigen Lichtquellen in beliebiger aber fest vorgegebener Reihenfolge aktiviert und deaktiviert werden.

Dabei können die breitbandigen Lichtquellen mit derselben Frequenz getaktet aktiviert und deaktiviert werden, wobei die Taktungen der einzelnen Weißlichtquellen zueinander phasenverschoben sind. Die Abfolge von farbigem Licht in den Messpunkten ist damit zeitlich zumindest versetzt.

Vorzugsweise wendet man eine zur Dispersionsrichtung korrelierende, beispielsweise eine proportionale, zumindest eine monoton steigende oder monoton fallende Phasenverschiebung an, wobei der Betrag der Phasenverschiebung dem gewünschten Dispersionsbereich angepasst ist. Unter der Voraussetzung, dass das über die Mikrolinsen-Optik in die Blendenöffnungen gelangende Licht von mindestens 2 benachbarten LEDs ausgeht, was unter Verwendung von sehr kleinen LEDs und einer entsprechenden Dimensionierung der Mikrolinsenoptik erreicht werden kann, ergibt sich ein praktisch kontinuierliches verändern der zentralen Wellenlänge des Lichtes welches durch die Blendenöffnung gelangt. Die Frequenz, mit welcher so die Lichtfarbe periodisch, kontinuierlich, und rampenförmig durchlaufen wird, entspricht der Taktfrequenz der breitbandigen Lichtquellen. Die Bildaufnahmeeinheit umfasst vorzugsweise ebenfalls ein Rasterelement, dessen Raster optisch äquivalent zum Raster der Einzellichtquellen angeordnet ist. Im einfachsten Fall wird dies durch ein exklusives Auslesen, bzw. Berücksichtigen der entsprechenden Bildpunkte des Bildaufnahmesensors realisiert, und in einem weniger einfachen aber ggf. notwendigen Fall ist dies eine Lochrasterblende, die ein Raster von Blendenöffnungen aufweist.

Vorzugsweise umfasst die Bildaufnahmeeinheit einen flächigen Bildaufnahmesensor, der zumindest an den Positionen, an welchen die Messpunkte abgebildet sind, eine zeitliche Diskriminierung der von den Messpunkten rückgestreuten Intensität erlaubt.

Vorzugsweise wird dies durch einen Lock-in-Kamera-Sensor möglich. Solche Lock-in-Kamera-Sensoren liefern am Ausgang prinzipiell neben dem Amplitudensignal für jeden Pixel zumindest auch noch ein Phasensignal für einen gegebenenfalls dem Photonenstrom aufgeprägten Wechselanteil.

Eine aufwändige Version eines solchen Lock-in-Kamera-Sensors mit erhöhter Empfindlichkeit und Aufnahmegeschwindigkeit und besserer Dynamik steht als pOCT-Sensor (parallel Optical low-Coherence Tomography Sensor) oder Fast-Lock-in-Sensor am Markt zur Verfügung. Eine einfachere Version eines solchen Lock-in-Kamera-Sensors ist mittlerweile als Time-of-Flight-Image-Sensor (ToF-Sensor), auch Distance-Aerea-Image-Sensor (DAI-Sensor) genannt, auf dem Markt kostengünstig erhältlich. Bei einem solchen ToF- oder DAI-Sensor werden für jedes Pixel jeweils mindestens zwei Speicherelemente zum Speichern einer erfassten Photonenintensität verwendet, wobei über eine Synchronisierungseinrichtung und/oder einen Triggeranschluss bestimmt wird in welches der Speicherelemente die momentan auftreffende Photonenintensität aufintegriert wird.

Ein solches Verhalten, dass die auftreffende Photonenintensität eines Messpunktes im Wechsel in verschiedenen Speicherelementen aufintegriert werden, lässt sich auch durch einen Full-Frame CCD-Sensor (FF-CCD), oder durch einen Frame-Transfer-CCD-Sensor (FT-CCD), oder durch einen Time-Delayed-Integration-Sensor (TDI) realisieren. Diese Sensoren arbeiten alle nach dem Prinzip, dass die durch die auftreffenden Photonen abgetrennten Ladungen zum Auslesen durch andere Aufnahmezellen (Charged-Coupled-Devices (CCD)) hindurch geleitet werden. Zur Realisierung der hier notwendigen Funktionalität müssen zwischen den auf dem Sensor abgebildeten Messpunkten in Richtung der Ladungskopplung des Sensors noch ausreichend unbelichtete CCD-Zellen zur Verfügung stehen die selbst kein Licht aufnehmen dürfen, also z.B. durch eine zu den Messpunkten passende Rasterblende abgedeckt sind. Dabei könnte auch ein weiteres feststehendes Mikrolinsenarray hilfreich sein um die für gewöhnlich mehr als 10 µm großen Blendenöffnungen der Lochrasterblende auf die Pixeldimension, welche für gewöhnlich kleiner als 10 µm ist, zu verkleinern. Wenn es gelingt die Rasterblendenmatrix und das Mikrolinsenarray so anzuordnen, dass jeder Messpunkt eine eigene Ladungskette, ohne Beeinflussung durch andere Messpunkte hat, so kann in vorteilhafter Weise die Ladungskette eines FF-CCD-Sensors oder TDI-Sensors kontinuierlich ausgelesen werden und sogar noch innerhalb der Auslesung die Integrationszeit durch Ändern der Taktfrequenz kontinuierlich geändert werden. Die ausgelesenen Bilder würden dann in den Spalten jeweils ein Hell-Dunkel-Signal tragen in dessen Phasenverschiebung relativ zum Auslesetakt die Zeitinformation beinhaltet ist.

Mit CCD- und TDI-Sensoren ist aber auch durch eine gezielte synchronisierte vor- und/oder rückwärtige Ladungsverschiebung während einer Bildintegration der gleiche Effekt wie im DAI / ToF-Sensor, allerdings mit verminderter Geschwindigkeit, darstellbar.

Bei all den genannten Lock-in-Kamera-Sensoren lassen sich aus den ausgelesenen Bilddaten ein Amplituden- und ein Phasensignal gewinnen. Dies ermöglicht eine zeitliche Diskriminierung der von den Messpunkten rückgestreuten Intensität. Die zeitliche Diskriminierung erfolgt dabei mit einer für das dreidimensionale Erfassen der Oberflächenstruktur ausreichend hohen Geschwindigkeit.

Alternativ kann die Bildaufnahmeeinheit einen CMOS-Bildwandler mit schneller Auslesemöglichkeit umfassen. CMOS-Bildwandler sind aufgrund ihrer Auslesestrategie prinzipiell geeignet, dass nur kleine Teilbereiche in entsprechend höherer Geschwindigkeit ausgelesen werden. Da bei der hier notwendigen Bildaufnahmeeinheit die Gesamtfläche aller Messpunkte auf dem Sensor nur weniger als 3%, im Extremfall sogar nur 0,1% der gesamten Bildfläche beträgt, kann der CMOS-Sensor bei geeigneter Ansteuerung und geeigneter Auslegung des Messpunkterasters, welches gegebenenfalls auch durch optische Abbildung oder durch Umleitung über optische Fasern erreicht werden kann, mit bis zu 1000facher Bildrate ausgelesen werden. Auch hiermit ist dann prinzipiell eine ausreichende zeitliche Diskriminierung bzw. eine Bestimmung der Phasenlage des Wechselanteils des ankommenden Photonenstroms möglich. Bei einer Umleitung des Ausleselichtes durch optische Fasern kann zudem besonders vorteilhaft auf beliebige kostengünstige oder schnelle Sensorformate, oder auch auf einen einfachen Zeilensensor, oder, da TDI-Sensoren meistens sehr schmal und länglich gebaut sind, auch auf solche kostengünstigere Standard-TDI-Sensoren zurückgegriffen werden.

Eine weitere Möglichkeit die zeitliche Diskriminierung mit einem gewöhnlichen Kamera-Sensor mit gewöhnlicher Bildrate zu erzielen, ist durch Anwendung einer schnellen Relativbewegung des aus den Blenden austretenden Lichtes über die Kamera-Sensoroberfläche. Vorzugsweise wird hierzu eine kreisförmige Bewegung des Lichtes der Blendenpunkte, mit einem maximalen Durchmesser etwas kleiner als dem minimalen Abstand der Messblenden entsprechend, durchgeführt. Hierzu kann z.B. der Kamera-Sensor, vorteilhafter Weise jedoch auch nur ein ca. 1:1 bis 1:2 abbildendes Mikrolinsenarray, kreisförmig mit einem Radius von beispielsweise 30 - 60µm unter der Aufnahmelochrasteblende mit einem ganzzahlig vielfachen der Bildwiederholrate, und einem ganzzahligen Bruchteil der Licht-Modulationsfrequenz um die Lichtachse schwingen. Der gleiche Effekt könnte durch eine zwischen der Aufnahmelochrasteblende und dem der Kamera-Sensor rotierenden Faserplatte mit schräg gestellten Fasern, oder über eine rotierende oder vibrierende Spiegelplatte oder über eine rotierende oder vibrierende durchsichtige Keilplatte, oder über ein mikromechanisches Spiegel-Array erreicht werden.

Eine geeignete zeitliche Diskriminierung des Photonenstroms in den Messpunkten ist nicht nur bei einem periodisch wellenförmig modulierten Photonenstrom in Sinus-, Dreieck- oder Rechteckform, sondern auch bei pulsförmig modulierten Photonenströmen möglich. Eine Diskriminierung eines pulsförmigen Signals ist vorzugsweise mit allen genannten CCD, TDI und CMOS-Sensoren möglich. Zur zeitlichen Diskriminierung von pulsförmigen Photonenströmen sind auch flächige Detektoren aus dem Gebiet der Röntgentechnik (Timepix-Detektoren) geeignet, die den direkten Eintrittszeitpunkt einzelner Photonenquanten mit sehr hoher zeitlicher Auflösung bestimmen.

Falls die ankommende Lichtmenge zu gering ist können alle genannten Sensoren gegebenenfalls zur Erhöhung der Empfindlichkeit durch vorgesetzte Lichtverstärkungssysteme wie Mikrokanal-Bildverstärker ergänzt werden. Bei den CCD-Sensoren sind hierfür EMCCD-Sensoren geeignet, welche bereits intern innerhalb der Ladungskette eine Ladungsmultiplikation durchführen.

Vorzugsweise ist der Triggeranschluss der oben genannten Bildaufnahmeeinheiten mit der Steuer- und Auswerteeinheit verbunden und diese dazu eingerichtet, den Triggeranschluss mit derselben Frequenz wie die breitbandigen Lichtquellen anzusteuern. Es ist aber auch möglich, dass die Bildaufnahmeeinheit ein Triggersignal zur Ansteuerung der Lichtquellen und des Mikrolinsenarrays sendet. Dadurch kann eine Korrelation der zeitlichen Abfolge innerhalb der Messeinrichtung hergestellt werden. Die Frequenz der Beleuchtungseinrichtung kann dabei zwischen dem 1-fachen und bis zum mehreren Millionenfachen der gewünschten Messpunkt-Bildrate betragen und wird im Wesentlichen durch den Bildwandler bestimmt.

Vorzugsweise ist die Steuer- und Auswerteeinheit ferner dazu eingerichtet, aus den von der Bildaufnahmeeinheit gelieferten Messpunktdaten den Abstand der Oberflächenstruktur zur Optik zu bestimmen. Beispielsweise kann bei einem DAI-Sensor die Steuer- und Auswerteeinheit dazu eingerichtet sein, den Abstand aus dem Verhältnis der in den beiden Speicherelementen eines Pixels erfassten Intensitäten zu bestimmen.

Unter chromatischer Optik wird in der vorliegenden Anmeldung eine Optik (auch nur eine Linse) verstanden, die eine chromatische Längsaberration CLA aufweist, die ausreicht, um mit den Wellenlängen des verwendeten Lichts den gewünschten Messbereich/Auflösung der Tiefeninformation über die Oberflächenstruktur abzudecken. Vorzugsweise ist daher die Optik eine hyperchromatische Optik, wodurch ein größerer Tiefenmessbereich erreicht wird. Eine hyperchromatische Optik hat eine äquivalente Abbesche Zahl, die kleiner ist als die Abbesche Zahl aller Einzelelemente, d.h. sie hat eine besonders hohe CLA. Vorzugsweise könnte die chromatische Optik durch eine elektrisch verstellbare Linse, vorzugsweise eine Flüssiglinse, ergänzt werden um einen ausreichenden durchgängigen Tiefenmessbereich zu gewährleisten.

Sämtliche obige Überlegungen zur Bildaufnahmeeinheit, gelten unabhängig von der Ausgestaltung der Lichtquelle auch für den erfindungsgemäßen Fall, dass anstatt der breitbandigen Lichtquellen mit dispersivem Element anderweitig Einzellichtquellen erzeugt werden, deren spektrale Zusammensetzung zeitlich variiert werden kann. Eine solche Lichtquelle besteht beispielsweise aus mehreren farbigen LEDs oder aus mehreren farbigen Laserlichtquellen, oder aus Weißlichtquellen mit nachgeschalteten Bandpass-Farbfiltern, welche mit geeigneten optischen Mitteln z. B. Prismen oder dichroitischen Spiegeln auf das Messpunktraster abgebildet werden und zeitlich individuell ansteuerbar sind. Erfindungsgemäß ist hierbei die Überlappung der Wellenlängenbänder dieser Lichtquellen, wobei schmale Lücken zwischen den Wellenlängenbändern dieser Lichtquellen auch noch durch eine entsprechende konfokale Unschärfe, oder durch die elektrisch verstellbare Linse, ausgeglichen werden können.

### KURZE BESCHREIBUNG DER ZEICHNUNGEN

Nachstehend wird die Erfindung anhand mehrerer Ausführungsbeispiele unter Bezugnahme auf die Zeichnung erläutert, wobei die Figuren 4-7 erfindungsgemäße Ausführungsbeispiele zeigen. Darin zeigt:
- Figur 1: einen schematischen Längsschnitt durch eine Intra-oralkamera zum dreidimensionalen Erfassen einer Oberflächenstruktur;
- Figur 2: eine perspektivische Darstellung einer Beleuchtungs- und Bildaufnahmeeinheit der Intraoralkamera;
- Figur 3: eine schematische Darstellung der Intraoralkamera, bei welcher die Bildaufnahmeeinheit einen Lock-In-Sensor und die Lichtquelle eine Weißlicht-LED-Zeile aufweist;
- Figur 4: eine perspektivische Darstellung einer weiteren Beleuchtungs- und Bildaufnahmeeinheit der Intraoralkamera;
- Figur 5: eine perspektivische Darstellung einer weiteren Beleuchtungs- und Bildaufnahmeeinheit der Intraoralkamera;
- Figur 6: eine schematische Darstellung der Intraoralkamera, bei welcher die Bildaufnahmeeinheit einen gewöhnlichen Kamera-Sensor aufweist und die Lichtquelle aus wenigen speziellen farbigen LEDs besteht;
- Figur 7: eine schematische Darstellung des Detektorpixels und der Ansteuersignale der Intraoralkamera, bei welcher die Bildaufnahmeeinheit einen Timepix-Detektor aufweist und die Lichtquelle kurze Lichtblitze aussendet;

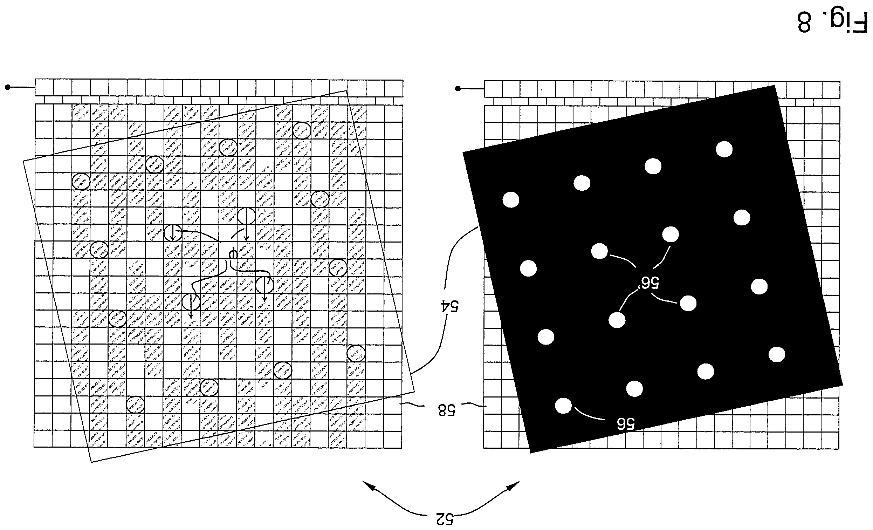

- Figur 8: eine schematische Darstellung der gewonnen Bilder einer Intraoralkamera, bei welcher die Bildaufnahmeeinheit einen CCD- oder einen TDI-Kamera-Sensor aufweist.

### DETAILLIERTE BESCHREIBUNG BEVORZUGTER ASUSFÜHRUNGSBEISPIELE

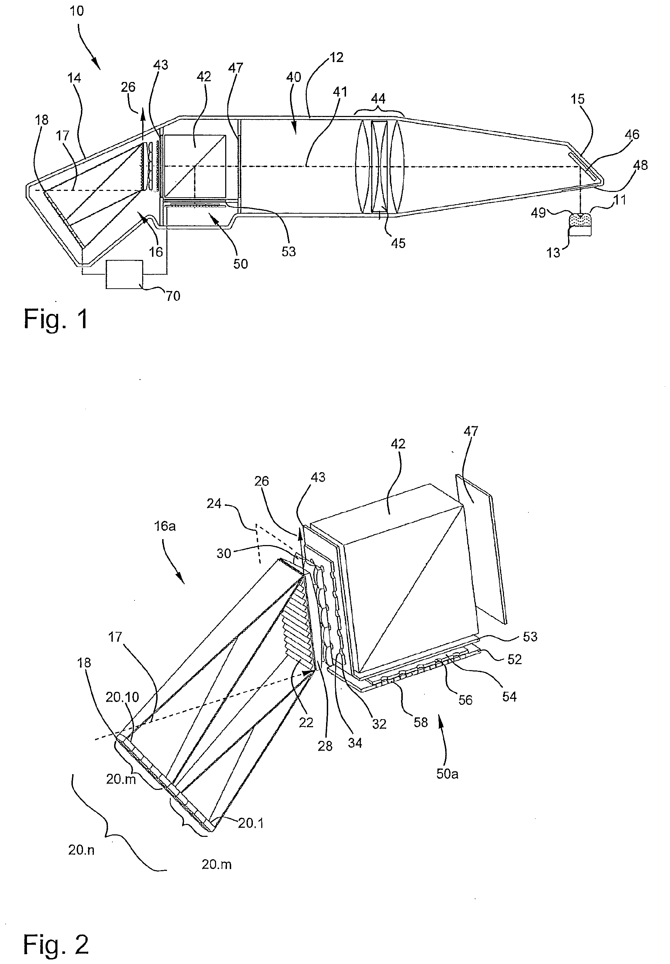

Figur 1 zeigt beispielhaft für eine Vorrichtung zum dreidimensionalen Erfassen einer Oberflächenstruktur eine Intra-oralkamera 10 zur Anwendung in der Dentalmedizin, insbesondere zum Erfassen von Zähnen 13 und deren Oberflächenstruktur 11.

Die Intraoralkamera 10 weist ein längliches Gehäuse 12 auf, welches die wesentlichen Komponenten der Intraoralkamera 10 aufnimmt.

Im Inneren des Gehäuses 12 ist an dessen proximalen Ende 14 eine Lichtquelle 16 angeordnet, deren detaillierter Aufbau besser aus Figur 2 ersichtlich ist.

Die Lichtquelle 16 weist zur Erzeugung des Beleuchtungslichts zunächst eine Zeile 18 von mehreren (hier zehn) nebeneinander angeordneten Weißlicht-LEDs 20.n (20.1 bis 20.10) als einzeln ansteuerbare Weißlichtquellen auf.

Ferner weist die Lichtquelle 16 als dispersives Element ein Gitter 22 mit einer Gitterebene 24 und einer Dispersionsrichtung 26 auf.

Die Zeile 18 und das Gitter 22 sind dabei derart versetzt zueinander angeordnet, dass für jede Weißlicht-LED 20.n die nullte Beugungsordnung des Gitters 22 nicht unter 0° senkrecht aus dem Gitter 22 austritt, sondern bereits einen Auslenkungswinkel aufweist. Insbesondere ist die Zeile 18 dabei so angeordnet, dass eine senkrechte Projektion der Zeile 18 auf die Gitterebene 24 zumindest eine Richtungskomponente in Dispersionsrichtung 26 aufweist. Vorzugsweise ist die Projektionsrichtung parallel zur Dispersionsrichtung 26. Hierbei ist die Zeile 18 aus Kostengründen jeweils geradlinig ausgeführt. Die theoretisch bevorzugte Gestalt der Zeile 18 wäre jedoch ein Kreisbogen, dessen Mittelpunkt im Zentrum des Gitters 22 liegt.

Aufgrund der Beugungswirkung des Gitters 22 werden die spektralen Anteile des von den Weißlicht-LEDs 20.n kommenden Beleuchtungslichts in Dispersionsrichtung 26 voneinander getrennt, wobei je nachdem welche Weißlicht-LED 20.n aktiviert ist, im Strahlengang nach dem Gitter 22 jeweils unterschiedlich farbiges Beleuchtungslicht erzeugt wird.

Im Strahlengang nach dem Gitter 22 sind ferner ein Mikrolinsenarray 30 und eine Beleuchtungslochrasterblende 32 so angeordnet, dass das vom Gitter 22 kommende, in 1. Ordnung gebeugte, farbige Beleuchtungslicht auf Blendenöffnungen 34 der Beleuchtungslochrasterblende 32 fokussiert wird.

Alle Weißlicht-LEDs 20.n der Zeile 18 sind dabei unterhalb der Strahlrichtung 17 der 0. Beugungsordnung der untersten Gitterlinien des Gitters 22 angeordnet, damit kein ungebeugtes weißes Licht durch die Blendenöffnungen 34 tritt. Zur besseren Ausnutzung des von den Weißlicht-LEDs 20.n ausgesendeten Lichtes befindet sich in diesem Strahlengang zudem eine Kondensorlinse 28, welche je nach Position als eine normale konvexe Linse oder als konvexe Zylinderlinse ausgeführt ist.

Im weiteren Verlauf des Strahlengangs folgt eine chromatisch-konfokale Optik 40 mit einer optischen Achse 41, entlang welcher ein Polarisationsfilter 43, ein Strahlteilerwürfel 42, eine Verzögerungsplatte 47 und eine hyperchromatische Linsengruppe 44 angeordnet sind. Durch ein weiteres, im zurücklaufenden Strahlengang vor der Bildaufnahmeeinheit 50 eingebautes Polarisationsfilter 53, kann Störlicht, welches nicht den vorgesehenen optischen Strahlengang durchlaufen hat, gemindert werden. Zu diesem Zweck wird als Strahlteilerwürfel 42 vorzugsweise auch ein polarisierender Strahlteilerwürfel 42 verwendet. ein Strahlteilerwürfel 42 und eine hypochromatische Linsengruppe 44 angeordnet ist. Ferner umfasst die Optik 40 am distalen Ende 15 der Intraoralkamera 10 einen Umlenkspiegel 46, mit welchem der Strahlengang auf ein seitlich am Gehäuse 12 angeordnetes Sichtfenster 48 umgelenkt wird.

Die Optik 40 ist derart ausgestaltet, dass die Blendenöffnungen 34 der Beleuchtungslochrasterblende 32 auf die zu erfassende Oberfläche 11 abgebildet werden. Die Blendenöffnungen 34 stellen so Einzellichtquellen dar, die auf der Oberflächenstruktur 11 ein Raster von beleuchteten Messpunkten 49 erzeugen. Die Beleuchtungslochrasterblende 32 und die Optik 40 sind dabei so aufeinander abgestimmt, dass die Messpunkte 49 lateral etwa zwischen 100 µm und 500 µm voneinander beabstandet sind, um ein Übersprechen zwischen den Messpunkten 49 zu verhindern.

Die Intraoralkamera 10 weist ferner eine Bildaufnahmeeinheit 50 auf, die optisch äquivalent zur Lichtquelle 16 neben dem Strahlteilerwürfel 42 angeordnet ist.

In den Figuren 1 bis 3 umfasst die Bildaufnahmeeinheit 50 einen Bildwandler 52 sowie eine Aufnahmelochrasterblende 54, die ebenfalls ein Raster von Blendenöffnungen 56 umfasst. In diesem Fall bedeutet eine optisch äquivalente Anordnung der Lichtquelle 16 und der Bildaufnahmeeinheit 50, dass die Optik 40 die Blendenöffnungen 34 der Beleuchtungslochrasterblende 32 und die Blendenöffnungen 56 der Aufnahmelochrasterblende 54 auf dieselben Messpunkte 49 auf der Oberflächenstruktur 11 abbildet.

Auf die Aufnahmelochrasterblende 54 wird vorzugsweise verzichtet, wenn der Bildwandler kein CCD-Sensor und die Pixel des Bildwandlers zumindest etwas kleiner sind, als die vorgesehenen Blendenöffnungen 56 sind. Die Funktion der Aufnahmelochrasterblende kann dann einfach durch unterlassene Berücksichtigung der normalerweise durch die Aufnahmelochrasterblende 54 abgedeckten Aufnahmepixel 58 des Bildwandlers 52 erfolgen.

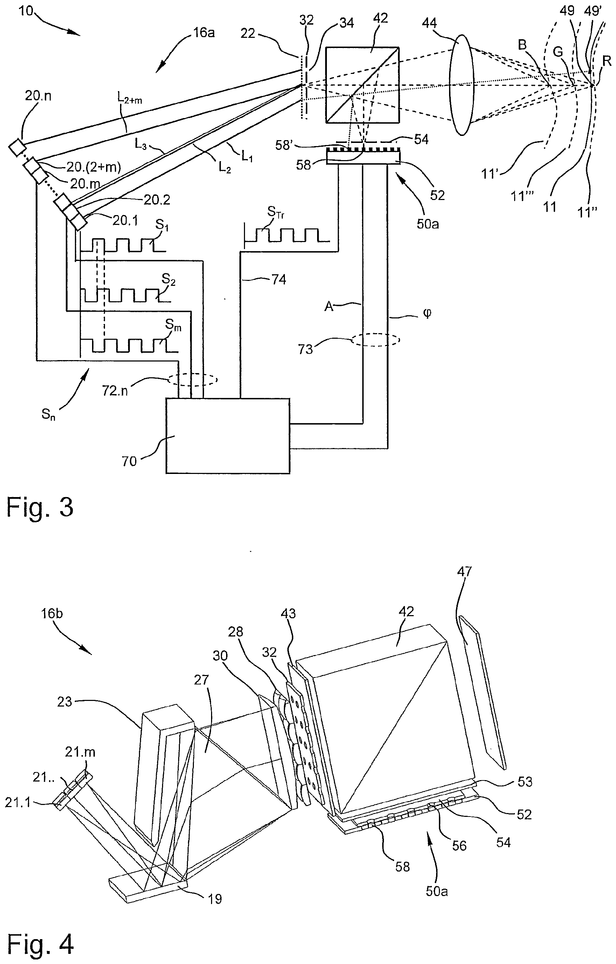

Als Bildwandler 52 ist ein Lock-in-Kamera-Sensor vorgesehen, wobei es lediglich von der geforderten Messgeschwindigkeit und Auflösung und dem vorhandenen Störlicht abhängig ist ob hier ein Fast-lock-in-Sensor (pOCT) oder ein Time-of-Flight-Sensor (DAI-Sensor) oder ein entsprechend betriebener CCD-Sensor oder sogar ein entsprechend ausgewerteter schneller CMOS-Sensor verwendet wird. Die hier wesentliche Eigenschaft der Bildaufnahmeeinheit 50 bzw. des Bildwandlers ist, dass jeder Pixel, der Licht an der Position der Blendenöffnungen 56 empfängt, einen Lock-in-Bearbeitungsmechanismus beinhaltet, und somit neben dem Amplitudensignal A auch noch die Phasenlage ϕ des Wechselanteils des Photonenstroms angibt (vgl. Figur 3). Je nach Typ des verwendeten Lock-in-Kamera-Sensors erfolgt die Ermittlung der Phasenlage ϕ jedoch auch erst in der Steuer- und Auswerteeinheit 70.

Schließlich umfasst die Intraoralkamera 10 noch eine Steuer- und Auswerteeinheit 70, die in Figur 1 zu Zwecken der besseren Darstellbarkeit außerhalb des Gehäuses 12 angeordnet ist. Selbstverständlich kann die Steuer- und Auswerteeinheit 70 jedoch auch vollständig oder teilweise innerhalb des Gehäuses 12 angeordnet sein.

Die Steuer- und Auswerteeinheit 70 ist zur Steuerung der Weißlicht-LEDs 20.n mit der Zeile 18 der Lichtquelle 16 verbunden. Obwohl eine solche Verbindung am realen System wohl über eine Digitalverbindung, eine Multiplexverbindung oder dergleichen realisiert ist, sind in Figur 3 zum besseren Verständnis jeweils einzelne Leitungen 72.n zu den entsprechenden Weißlicht-LEDs 20.n gezeigt. Dies gilt generell für hier gezeigte Verbindungen.

Eingangsseitig ist die Steuer- und Auswerteeinheit 70 über eine Ausleseleitung 73 mit dem Bildwandler 52 verbunden, wobei in dieser Ausleseleitung 73 das Phasensignal ϕ und das Amplitudensignal A, oder zumindest Rohsignale die zur Berechnung des Phasen- und Amplitudensignals geeignet sind, übertragen wird.

Ferner ist die Steuer- und Auswerteeinheit 70 über eine Triggerleitung 74 mit der Bildaufnahmeeinheit 50a verbunden, welche den Nullpunkt der Phasenlage überträgt.

Nicht gezeigt ist eine Verbindung der Steuer- und Auswerteeinheit 70 zu einem üblicherweise vorhandenen Anzeigegerät, meist ein Computer mit einem Monitor, der zugleich eine Berechnung der vollständigen Oberflächenstruktur aus den einzelne Aufnahmen, eine Speicherung und eine messtechnische Auswertung der Aufnahmen.

Die Intraoralkamera 10 arbeitet wie folgt:
Mit Hilfe der Steuer- und Auswerteeinheit 70 werden die Weißlicht-LEDs 20.n über die Leitungen 72.n mit Ansteuersignale S.n beaufschlagt.

Vorzugsweise werden hier Ansteuersignale verwendet, die zu einer sinus-, dreieck- oder rechteckförmigen Lichtemission führen. In Figur 3 sind beispielsweise Rechtecksignale dargestellt, welche, wie ersichtlich, zueinander jeweils derart Phasenverschoben sind, dass jede Weißlicht-LED 20.n ungefähr eine Phasenverschiebung von maximal ϕ/m zur nächsten LED verschoben ist. ϕ ist hierbei der prinzipbedingte Phasenmessbereich des verwendeten Bildaufnahmesensors welcher meistens zumindest π (180° bzw. +/-90°) und maximal 2π (360° bzw. +/-180°) beträgt, und m ist hierbei die Anzahl (vgl. 20.m in Figur 2) der LEDs die notwendig ist um den für den Tiefenmessbereich notwendigen Wellenlängenbereich in der Dispersionseinrichtung darzustellen. Die Gesamtanzahl n der LEDs ist also größer als m, da die Beleuchtungslochrasterblende 32 auch in Dispersionrichtung 26 ausgedehnt ist, und auch die am Rand liegenden Blendenöffnungen 34 den gesamten Wellenlängenbereich übertragen sollen. Die LED 20.x könnte also das gleiche Ansteuersignal wie die LED 20.(x+m) und ggf. 20.(x+2m) erhalten. Die Frequenz der Ansteuersignale Sn ist hingegen für alle LEDs 20.n gleich und wird erheblich durch den verwendeten Bildaufnahmesensor bestimmt. Bei ToF, bzw. DAI-Sensoren liegt sie im Bereich von 500 kHz bis 40 MHz. Bei pOCT-Sensoren ist der gewöhnliche Arbeitsbereich zwischen 1 kHz und 100 kHz. Wird die Lock-in-Funktion durch einen der genannten geeigneten CCD-Sensoren dargestellt können prinzipiell Frequenzen im Bereich von 1kHz bis 50MHz verwendet werden. Falls das Phasensignal unter Verwendung eines CMOS-Sensors gewonnen wird beträgt die Frequenz vorzugsweise maximal einen Bruchteil der maximalen Bildausleserate des Sensors, also weniger als 1kHz. Die Frequenz sollte jedoch mindestens ein Vielfaches der gewünschten Messpunktabtastrate betragen, also mindestens 100Hz.

Aufgrund dessen, dass jede Weißlicht-LED 20.n unter einem anderen Winkel zum Gitter 22 bezüglich der Dispersionsrichtung 26 steht und die unterschiedlichen spektralen Anteile des einfallenden Lichts am Gitter 22 unterschiedlich stark gebeugt werden, wird jede Blendenöffnungen 34 der Beleuchtungslochrasterblende 32 in zeitlicher Abfolge von unterschiedlich farbigem Beleuchtungslicht beleuchtet. Dadurch erscheinen die Blendenöffnungen 34 als Einzellichtquellen, die in zeitlicher Abfolge ihre Farbe ändern.

Da an den Blendenöffnungen 34 das austretende Licht für jede Lichtfarbe eine definierte Phasenlage besitzt und durch die konfokale Diskriminierung in der Aufnahmelochrasterblende 54 nur Photonen eines schmalen Wellenlängenbandes in den jeweiligen Pixel 58 gelangt, korreliert die Phasenlage des Pixelsignals der Bildaufnahmeeinheit 50a direkt mit dem Abstand des Messpunktes von der Objektivlinse.

Für die zeitliche Abfolge sind grundsätzliche beliebige Muster denkbar. Die Reihenfolge innerhalb der Abfolge muss jedoch festgelegt sein, damit eine Korrelation mit den Daten der Bildaufnahmeeinheit möglich ist.

Über den Strahlteiler 42 und die hyperchromatische Linsengruppe 44 werden die Einzellichtquellen auf die Oberflächenstruktur 11 des Zahnes 13 abgebildet.

Aufgrund der erhöhten chromatischen Längsabberation der hyperchromatischen Linsengruppe 44 werden die Einzellichtquellen jedoch je nach momentaner Farbe in unterschiedlicher Entfernung von der Linsengruppe 44 fokussiert. Daher wird jeweils nur dann eine Einzellichtquelle scharf in einen Messpunkt 49 auf der Oberflächenstruktur 11 abgebildet, wenn die Entfernung der Oberflächenstruktur 11 mit der Fokussierungsentfernung der jeweiligen Farbe übereinstimmt. Die anderen Farben werden über einen größeren lateralen Bereich ausgeschmiert, wodurch sie nur noch zu einem kleinen Teil durch die Optik 40 zurück, durch die gegebenenfalls zugehörige Messblende 56, in den zugehörigen Aufnahmepixel 58 des Bildwandlers gelangen.

Beispielsweise wird bei typischen hypochromatischen Linsengruppen 44, wie in Figur 3 gezeigt, blaues Licht B näher zur Linsengruppe hin fokussiert als rotes Licht R. Verläuft die Oberflächenstruktur 11 also wie in Figur 3 als gestrichelte Linie 11' gezeigt, so erscheint der beleuchtete Lichtpunkt zu jenem Zeitpunkt scharf, zu welchem von der Einzellichtquelle her blaues Licht B einfällt. Umgekehrt wäre bei der gestrichelt dargestellten Linie 11" der Zeitpunkt des roten Lichts R maßgeblich.

Das von der Oberflächenstruktur 11 zurückgestreute Licht eines scharfen Messpunktes 49 fällt dann wieder weitestgehend derart in die Optik 40 der Intraoralkamera 10, dass der Messpunkt 49 aufgrund des Strahlteilerwürfels 42 in die zugehörige Blendenöffnung 56 bzw. den zugehörigen Aufnahmepixel 58 der Bildaufnahmeeinheit 50a abgebildet wird.

Um die erforderliche Tiefenauflösung senkrecht zur Oberflächenstruktur 11 zu erhalten, kommt nun der Lock-In-Kamera-Sensor als Bildwandler 52 zum Einsatz.

Wie anhand der durchgezogenen Linie 11 in Figur 3 erkennbar ist, hat die Oberflächenstruktur 11, in dem Messpunkt 49, der zu dem betrachteten Pixel 58 des Lock-in-Kamera-Sensors gehört, einen Abstand zur Optik 40, bei welchem die chromatisch konfokale Bedingung am ehesten für die Lichtfarbe Rot erfüllt ist, welche für diese laterale Messpunktposition durch die Beugung 1. Ordnung am Gitter 22 nur im Lichtstrahl L2 aus der LED 20.2 stammen kann. Das detektierte Lichtsignal im Pixel 58 hat somit die Phasenlage der Leuchtdiode 20.2. Für den Messpunkt 49' stammt diese Lichtfarbe hingegen aus dem Lichtstrahl L1 aus der Leuchtdiode 20.1. und trägt somit deren Phasensignal. Wäre die Oberfläche 11 hingegen durch die Linie 11' dargestellt, so würde das Phasensignal des Pixels 58 durch den Lichtstrahl L2+m aus der LED 20.m+2 bestimmt, da nur deren blauer Lichtanteil durch das Gitter in 1. Ordnung in diese Richtung gebeugt wird, und die chromatisch konfokale Bedingung im Punkt B erfüllt ist. Zunächst scheint durch diese Funktionsweise theoretisch nur die Messung von m Tiefenstufen möglich zu sein, da die Lichtstrahlen L, welche den Tiefenmessbereich abdecken, ja nur aus LEDs stammen die im Winkelbereich der 1. Beugungsordnung zwischen den Lichtwellenlängen des konfokalen Tiefenbereichs der hyperchromatischen Linsengruppe liegen. Aufgrund der endlichen Kleinheit der Blenden 34 und 56 gibt es jedoch einen fließenden Übergang zwischen diesen theoretischen Tiefenstufen. So wird im Falle des Messpunktes 49 auch eine kleine Lichtmenge aus der LED 20.3 über den Lichtstrahl L3 zur Beleuchtung des Pixels 58 beitragen, da auch dieses Licht sogar über den gleichen Beugungswinkel am Rand der Blende 34 noch eine identische Wellenlänge zum Messpunkt 49 sendet. Zusätzlich gelangt wegen der Kegelform der farbigen Lichtstrahlen und der endlichen Kleinheit der Messblende 56 auch noch ein nennenswerter Anteil des Lichtes mit benachbarten Wellenlängen in den Aufnahmepixel 58. Dieses Licht stammt dann ebenfalls zu einem kleinen Anteil aus den benachbarten LEDs. Die hinter der Messblende 56, bzw. im Aufnahmepixel 58 gemessene Phase entspricht somit einer Überlagerung des Phasensignals aus mindestens 2 benachbarten LEDs der LED-Zeile 18 und ändert sich kontinuierlich mit dem Abstand des Messpunktes 49.

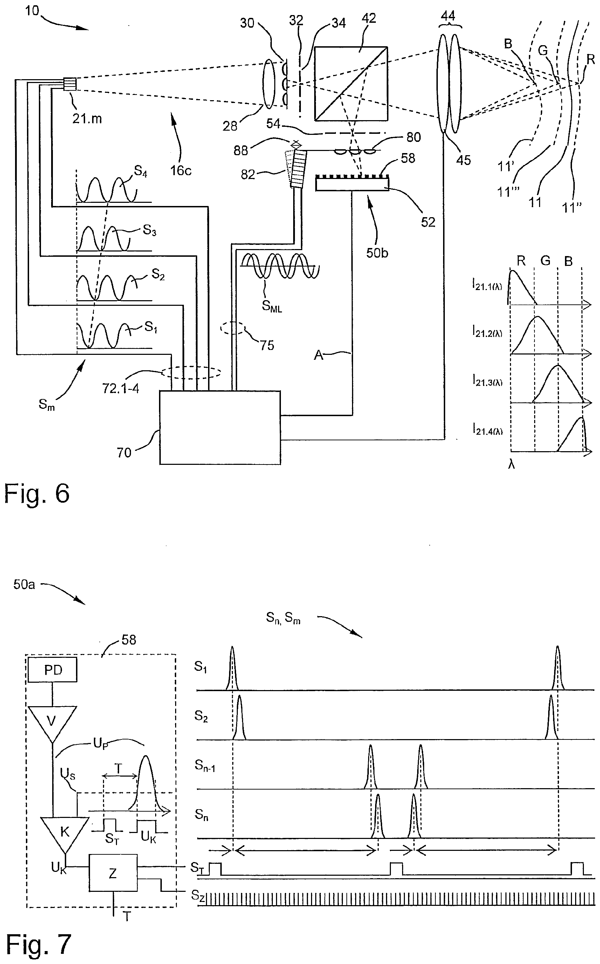

Analog hierzu ist in Figur 7 dargestellt wie die Lichtquellen (16, 16a, 16b, 16c) für einen Betrieb mit überlagerten pulsförmigen Lichtblitzen durch die Signale Sₙ, Sₘ beispielsweise angesteuert werden können, um einen Timepix-Detektor als Bildaufnahmesensor 52 in der Bildaufnahmeeinheit 50a zu verwenden. In Figur 7 ist ein einzelner Aufnahmepixel 58 des Timepix-Detektors (T-Pixel) dargestellt. Der ankommende überlagerte Lichtblitz trägt die Tiefeninformation in seiner relativen Ankunftszeit T. Die Photodiode PD und der Verstärker V des T-Pixels erzeugen einen Spannungspuls U_{P} welcher im Komperator K mit der Schwellenspannung Us verglichen wird. Der durch das Triggersignal S_{T} gestartete Zähler zählt die Impulse des Zeitsignals Sz bis er durch das Komperatorsignal U_{K} gestoppt wird und liefert so als Ergebnis die relative Ankunftszeit T des überlagerten Lichtblitzes. Da der T-Pixel gegebenenfalls nicht gleichzeitig die Amplitude oder den Zeitpunkt der Unterschreitung der Schwellenspannung U_{P} detektiert ist es gegebenenfalls Notwendig einen 2. Messvorgang mit umgekehrte Impusfolge der Signale Sₙ, Sₘ durchzuführen, um die Tiefenmessung nicht zu sehr durch Amplitudenunterschiede der in den Messpunkten 49 gestreuten Lichtblitze zu verfälschen. Vorzugsweise sind die Steuerimpulse Sₙ, Sₘ sinusförmig und zum nächsten Nachbar jeweils um 2/3π in ihrer Phase verschoben. Bei geeigneter Dimensionierung der Blenden 34 und 54 und bei geeigneter Positionierung und Dimensionierung der LEDs sind dann, ebenso wie bei der Verwendung symmetrischer Ansteuerung, auch Abständeermittelbar, die nicht einer exakten Fokuslage entsprechen.

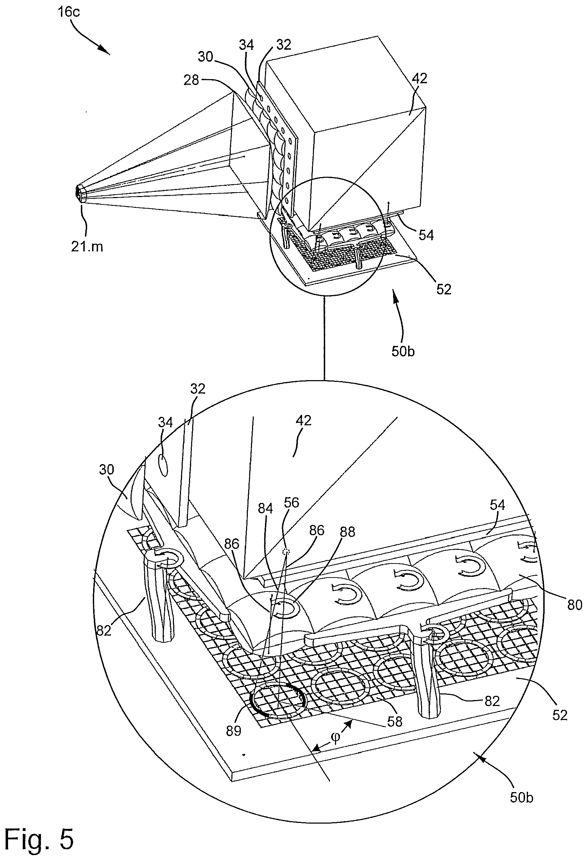

Figur 4 zeigt eine weitere chromatisch modulierte Lichtquelle die an der in Figur 1 dargestellten Intraoral-3D-Kamera 10 verwendet werden kann. Bei dieser Lichtquelle 16b wird ein Konzentrator 27 verwendet der lediglich notwendig ist um die bereits in den Lichtquellen 21.1 bis 21.3 auf ein begrenztes, verschiedenfarbiges, breites, Wellenlängenband eingegrenztes Farbspektrum zusammenzuführen und zentral durch die Blendenöffnungen 34 des Blendenrasters 32 zu führen. Der Konzentrator 27 ist dann vorzugsweise ein dispersives Element und hier als Prisma ausgeführt. Zur Umlenkung und Verlängerung des Strahlengangs wird vorzugsweise noch ein Umlenkspiegel 19 und eine Blende 23 zur Unterdrückung des direkt in Richtung strahlenden Lichtes eingebaut. Der Konzentrator 27, sowie der Umlenkspiegel 19 und die Blende 23 können dabei auch ganz entfallen wenn die Lichtquellen 21.1 bis 21.m extrem klein ausgeführt sind, sodass die Abbildung jeder Lichtquelle praktisch vollständig durch die Blendenöffnungen 34 in jeden zugehörigen Messpunkt, und zurück durch die Blendenöffnungen 56 in die Bildaufnahmeeinheit gelangt. Eine solche Lichtquelle 16c ist in Figur 5 dargestellt. Vorzugsweise werden in den Lichtquellen 16b und 16c nur wenige, vorzugsweise zwischen 3 und 7, Einzellichtquellen 21.1 bis 21.3 bzw. 21.4 verwendet, deren Wellenlängenspektrum zumindest eine sinus- oder dreiecksähnliche Form besitzen. Zusätzlich müssen sich diese Wellenlängenspektren jeweils bis ca. zu ihrer mittleren Wellenlänge gegenseitig überlappen. In Figur 6 sind Beispielsweise solche bevorzugt verwendeten Wellenlängenspektren I_{21.1(λ)} bis I_{21.4(λ)} für eine Lichtquelle 16b oder 16c mit 4 Einzellichtquellen und die zugehörigen Ansteuersignale S₁ bis S₄ dargestellt. Angesteuert werden diese Einzellichtquellen ebenfalls mit einem periodischen Signal gleicher Frequenz, welches zu einer sinus- oder dreiecksähnlichen Photonen-Intensitätsmodulation führt. Die Modulation erfolgt ebenfalls phasenversetzt. Entsprechend der kleineren Anzahl von z. B. m=4 Lichtquellen 21.1 bis 21.4 ist der Verschiebungswinkel der Signale S₁ bis S₄ deutlich größer. Er hat vorzugsweise den Wert 2π/(m+1). Das durch die Blendenöffnungen 34 der Beleuchtungslochrasterblende 32 gelangende Licht ist dann prinzipiell nahezu weißes Licht, da über eine Ansteuerperiode hinweg alle Wellenlängen ausgesendet werden. Genau betrachtet hat aber jede Wellenlänge Durch Überlagerung der beteiligten Lichtquellen eine eigene Phasenlage die sich kontinuierlich zur Wellenlänge verhält.

Das in der Bildaufnahmeeinheit 50 detektierte Signal hat jetzt eine alleinig durch Überlagerung der durch die konfokale Bedingung gefilterten schmalen Farbanteile aus zumindest 2 der überlappenden Spektren I_{21.m(λ)} bestimmte Phasenlage, welche ebenso mit der Tiefe des Messpunktes korreliert.

Eine einzelne Lichtquelle des Ausführungsbeispiels 2 mit einem vorzugsweise verwendeten Wellenlängenspektrum I_{21.m(λ)} kann beispielsweise aus einer farbigen LED, welche bereits ein ausreichendes Wellenlängenband emittiert, oder beispielsweise aus einer blau emittierenden LED oder Laserdiode mit einem nachgeschalteten fluoreszierenden Farbstoff welcher ein entsprechendes Wellenlängenband emittiert, oder aus einer breitbandig emittierenden weißen LED mit einem nachgeschalteten Farbfilter, welcher ein entsprechendes Wellenlängenband durchlässt, hergestellt werden.

Da mit den verfügbaren Wellenlängenspektren von Leuchtdioden und den verfügbaren Floureszenzfarbstoffen, und auch mit Farbfiltern in der Praxis jedoch nicht jedes Wellenlängenspektrum darstellbar ist, und auch die Objekte nicht jede Wellenlänge gleich gut zurück streuen könnten, insbesondere mit solchen Lichtquellen 21.m, schlecht messbare Zonen oder Lücken im Tiefenmessbereich entstehen. Zur Behebung dieses Mangels ist in der chromatischen Linsengruppe 44 eine elektrisch verstellbare Linse 45 eingefügt, welche durch eine entsprechende Ansteuerung durch die Steuereinheit 70 eine passende Verschiebung des Tiefenmessbereichs bewirkt. Vorzugsweise wird hierbei eine stufige Ansteuerung, welche mit dem Bildaufnahmezyklus des Kamera-Sensors 52 synchronisiert ist verwendet. Es ist jedoch auch möglich die Linse 45 periodisch anzusteuern, dann muss jedoch die Steuerfrequenz der Lichtquellen 20.n, 21.m ein ganzzahlig vielfaches diese periodischen Signals betragen.

Zur Detektion können hier ebenfalls die Lock-in-Kamera Sensorprinzipien verwendet werden. Eine pulsförmige Modulation der Photonenintensität ist hier auch prinzipiell möglich jedoch nicht sinnvoll, da dann zumindest auch noch die Intensitätsverhältnisse des ankommenden Photonenstromes ausgewertet werden müssten.

In Figur 5 und Figur 6 ist jedoch für das Ausführungsbeispiel 2 eine Bildaufnahmeeinheit 50b dargestellt, bei welcher ein gewöhnlicher Kamera-Sensor 52 verwendet werden kann. Zur zeitlichen Diskriminierung der in den Messblenden 56 ankommenden Photonenströme werden die Messblenden über ein korrespondierendes Mikrolinsenarray 80, welches durch einen Antrieb 82, vorzugsweise ein Piezoschwinger oder ein magnetisches Schwingsystem, in eine kreisende Schwingbewegung 88 versetzt wird, auf den gewöhnlichen Kamera-Sensor 52 abgebildet. Bei einem vorzugsweise verwendeten leicht vergrößernden Abbildungsmaßstab von ca. 1:1 bis 1:2 entsteht auf dem Kamera-Sensor 52 an jeder Messpunktposition eine kleine ringförmige Belichtung 89, deren Radius ca. doppelt bis 3fach so groß ist wie die Schwingbewegung 88 des Mikrolinsenarrays 80. Die Frequenz für die Lichtquellensignale S₁ bis Sₘ muss dabei ein ganzzahlig vielfaches der Schwingfrequenz des Mikrolinsenarrays S_{ML} betragen. Der ringförmigen Belichtung 89 ist dann eine Modulation entsprechend der Phasenlage der ankommenden konfokal diskriminierten Lichtfarbe aufgeprägt. Aus dem Winkel ϕ dieses Ringmusters lässt sich somit die Tiefe des zugehörigen Messpunktes bestimmen. Im in Figur 5 abgebildeten Beispiel wurde als Steuerfrequenz für die Lichtquellensignale S die doppelte Frequenz der Schwingfrequenz des Mikrolinsenarrays S_{ML} gewählt.

## Patentansprüche

1. Vorrichtung (10) zum dreidimensionalen Erfassen einer Oberflächenstruktur (11; 11'; 11"; 11'''), insbesondere zum Erfassen von Zähnen (13),
a) mit einer chromatisch-konfokalen Optik (40, 42, 44), die eine optische Achse (41) umfasst, und
b) mit einer Lichtquelle (16; 16a; 16b; 16c), die ein Raster von lateral zur optischen Achse (41) beabstandeten Einzellichtquellen (34) aufweist,
c) wobei die Optik (40, 42, 44) die Einzellichtquellen (34) derart auf die Oberflächenstruktur (11; 11'; 11"; 11''') abbildet, dass dort ein Raster von Messpunkten (49) erzeugt wird, die voneinander lateral beabstandet sind,
d) wobei eine Bildaufnahmeeinheit (50; 50a; 50b) optisch so zu den Einzellichtquellen (34) angeordnet ist, um das Raster von Messpunkten (49) zu erfassen,
wobei
e) eine Steuer- und Auswerteeinrichtung (70) vorgesehen ist, die dazu eingerichtet ist,
- die Lichtquelle (16; 16a; 16b; 16c) derart anzusteuern, dass die Einzellichtquellen (34) in zeitlicher Abfolge Licht mit unterschiedlicher spektraler Zusammensetzung abgeben, und
- mit Hilfe der Bildaufnahmeeinheit (50; 50a; 50b) das Raster von Messpunkten (49) korreliert mit der zeitlichen Abfolge des von den Einzellichtquellen (34) abgegebenen Lichts zu erfassen,
**dadurch gekennzeichnet, dass**
die Lichtquelle (16b, 16c) vor einem Rasterelement (30, 32) zum Erzeugen der Einzellichtquellen (34) zumindest zwei breitbandige, einzeln ansteuerbare Lichtquellen (21.m) aufweist,
die derart klein ausgeführt sind, dass die Abbildung jeder Einzellichtquelle praktisch vollständig auf denselben Messpunkt abgebildet wird, und
deren Wellenlängenspektrum jeweils eine sinus- oder dreiecksförmige Intensitätsverteilung aufweist, welche sich zumindest teilweise überlappen, sodass durch eine periodische phasenversetzte sinus- oder dreiecksförmige Ansteuerung der ansteuerbaren Lichtquellen (21.m) eine periodische Spektrumsverschiebung des Lichtes der Einzellichtquellen (34) durch Überlagerung des Lichtes der breitbandige, einzeln ansteuerbaren Lichtquellen (21.m) erfolgt.

2. Vorrichtung nach einem der vorhergehenden Ansprüche, **dadurch gekennzeichnet, dass** die chromatische Optik (40) polarisierende Komponenten (42,43,47,53) enthält, derart eingebaut, dass im Wesentlichen nur das von der Oberfläche 11 gestreute, und aus der Lichtquelle (16, 16a, 16b) stammende Licht in die Bildaufnahmeeinheit (50, 50a, 50b) gelangt.

3. Vorrichtung nach einem der vorhergehenden Ansprüche, **dadurch gekennzeichnet, dass** die chromatische Linsengruppe (44) eine hyperchromatische Linsengruppe ist.

4. Vorrichtung nach einem der vorhergehenden Ansprüche, **dadurch gekennzeichnet, dass** die chromatische Linsengruppe (44) eine elektromechanisch verstellbare Linse (45) enthält, derart gestaltet, dass Lücken im Tiefenmessbereich, welche durch unzureichende Wellenlängenbänder der Lichtquellen (20.n, 21.m) entstehen überbrückt werden.

5. Vorrichtung nach Anspruch 1, **dadurch gekennzeichnet, dass** die einzeln ansteuerbaren Lichtquellen (20.n, 21.m) in beliebiger aber fest vorgegebener Reihenfolge angesteuert werden.

6. Vorrichtung nach einem der vorhergehenden Ansprüche, **dadurch gekennzeichnet, dass** die einzeln ansteuerbaren breitbandigen Lichtquellen (20.n, 21.m) mit derselben Frequenz eines nahezu zeitlich symmetrischen Hell-Dunkel-Steuersignals angesteuert werden, wobei die Steuersignale der Lichtquellen (20.n, 21.m) zueinander phasenverschoben sind.

7. Vorrichtung nach einem der vorhergehenden Ansprüche, **dadurch gekennzeichnet, dass** die elektromechanisch verstellbare Linse (45) eine Flüssiglinse ist, welche durch die Steuereinheit mit den Lichtquellen (20.n, 21.m) korreliert wird.

8. Vorrichtung nach einem der vorhergehenden Ansprüche, **dadurch gekennzeichnet, dass** über eine Synchronisationsleitung (74) die Steuereinrichtung (70) und die Bildaufnahmeeinheit (50, 50a) in Verbindung stehen um die einzeln ansteuerbaren breitbandigen Lichtquellen (20.n, 21.m) mit der Abtastfrequenz der Bildaufnahmeeinheit zu synchronisieren.

9. Vorrichtung nach einem der Ansprüche 1 bis 8, **dadurch gekennzeichnet, dass** die Bildaufnahmeeinheit eine Aufnahmelochrasterblende (54) mit optisch äquivalent zu den Lichtquellen (34) angeordneten Lochblenden (56) enthält, welche durch ein Array aus optischen Fasern auf eine beliebige Anordnung der Detektorpixel (58) umleitet.

10. Vorrichtung nach einem der vorhergehenden Ansprüche, **dadurch gekennzeichnet, dass** die Bildaufnahmeeinheit eine Aufnahmelochrasterblende (54) mit optisch äquivalent zu den Lichtquellen (34) angeordneten Lochblenden (56) enthält, welche durch ein lateral bewegliches Mikrolinsenarray (80) auf einen beliebigen Bildaufnahmesensor (52) abgebildet wird, wobei das lateral bewegliches Mikrolinsenarray (80) zumindest in einer Richtung durch einen Antrieb (82) in eine periodische Bewegung (88) versetzt wird, wobei die Ansteuerfrequenz (Sn, Sm) der Lichtquellen (20.n, 21.m) ein ganzzahlig vielfaches der zur Ansteuerfrequenz (S_{ML}) des Mikrolinsenarrays beträgt, und die Ansteuersignale der Lichtquellen (Sn, Sm) mit dem Ansteuersignal (S_{ML}) des Mikrolinsenarrays durch die Steuereinheit (70) korreliert wird, .

11. Vorrichtung nach einem der Ansprüche 1 bis 10, **dadurch gekennzeichnet, dass** die Bildaufnahmeeinheit (50, 50a) einen Fast-Lock-In-Kamera-Sensor (pOCT-Sensor) umfasst, bei welchem jedes Pixel (58) ein Amplitudensignal (A) und ein Phasensignal (ϕ) des ankommenden Photonenstroms ausgibt, welches direkt mit einer Lock-In-Auswerteschaltung im Pixel 58 gewonnen wird.

12. Vorrichtung nach einem der Ansprüche 1 bis 11, **dadurch gekennzeichnet, dass** die Bildaufnahmeeinheit (50, 50a) einen Time-Of-Flight-Kamera-Sensor (TOF- oder DAI-Sensor) umfasst, bei welchem jedes Pixel (58) mehrere Amplitudensignale ausgibt woraus sich das Phasensignal des ankommenden Photonenstroms durch die Steuereinheit (70) berechnen lässt.

13. Vorrichtung nach einem der Ansprüche 1 bis 9, **dadurch gekennzeichnet, dass** die Bildaufnahmeeinheit (50, 50a, 50b) einen CMOS-Kamera-Sensor umfasst, bei welchem das Phasensignal der Messpunkte durch Auswertung des Amplitudenbildes welches durch schnelles, ausschließliches Auslesen der durch die Messpunkte 56 belichteten Pixel gewonnen wurde, durch die Steuereinheit berechnet wird.

14. Vorrichtung nach einem der Ansprüche 1 bis 8 und 10, **dadurch gekennzeichnet, dass** die Bildaufnahmeeinheit (50b) einen CMOS-Kamera-Sensor umfasst, bei welchem das Phasensignal der Messpunkte durch Auswertung des Amplitudenbildes (89) welches durch eine laterale Bewegung (88) des Mikrolinsenarrays (80) gewonnen wurde, durch die Steuereinheit berechnet wird.

15. Vorrichtung nach einem der Ansprüche 1 bis 9, **dadurch gekennzeichnet, dass** die Bildaufnahmeeinheit (50, 50a, 50b) einen Impulse detektierenden Strahlungsbildsensor (Timepix Detektor) umfasst, bei welchem in jedem Pixel die Ankunftszeit eines Pulses gemessen wird, wobei hierfür kurze, intensive Steuersignale (Sₘ, Sₘ) welche vorzugsweise sinusförmigem Verlauf der Lichtblitze erzeugen, und welche weiterhin vorzugsweise zum nächsten Nachbar jeweils um 2/3π in ihrer Phase verschoben sind, verwendet werden, und die Tiefeninformation aus den Ankunftszeiten der Lichtblitze durch die Steuereinheit berechnet wird.

## Claims

1. Device (10) for the three-dimensional detecting of a surface structure (11; 11'; 11"; 11'''), in particular for detecting teeth (13),
a) with a chromatic-confocal optics (40, 42, 44) comprising an optical axis (41), and
b) with a light source (16; 16a; 16b; 16c) which has a raster of individual light sources (34) spaced laterally to the optical axis (41),
c) wherein the optics (40, 42, 44) images the individual light sources (34) onto the surface structure (11; 11'; 11"; 11''') such that a raster of measurement points (49), which are laterally spaced apart from one another, is generated there,
d) wherein an image recording unit (50; 50a; 50b) is optically arranged with respect to the individual light sources (34) in such a way to detect the raster of measurement points (49)
wherein
e) a control and evaluation device (70) is provided, which is configured
- to control the light source (16; 16a; 16b; 16c) such that the individual light sources (34) emit light of different spectral composition in temporal sequence, and
- to detect the raster of measurement points (49) correlated with the temporal sequence of the light emitted by the individual light sources (34) with the aid of the image recording unit (50; 50a; 50b),
**characterized in that**
the light source (16b, 16c) comprises at least two broadband, individually controllable light sources (21.m) before a raster element (30, 32) for generating the individual light sources (34),
which are configured so small that the image of each individual light source is imaged practically completely on the same measurement point, and
whose wavelength spectrum in each case has a sinusoidal or triangular intensity distribution which at least partially overlap, so that by a periodic phase-offset sinusoidal or triangular control of the controllable light sources (21.m) a periodic spectral shift of the light of the individual light sources (34) occurs by superimposition of the light of the broadband, individually controllable light sources (21.m).

2. Device according to one of the preceding claims, **characterized in that** the chromatic optics (40) comprises polarizing components (42, 43, 47, 53) installed such that substantially only the light scattered by the surface 11 and coming from the light source (16, 16a, 16b) enters the image recording unit (50, 50a, 50b).

3. Device according to one of the preceding claims, **characterized in that** the chromatic lens group (44) is a hyperchromatic lens group.

4. Device according to one of the preceding claims, **characterized in that** the chromatic lens group (44) comprises an electromechanically adjustable lens (45) configured such that gaps in the depth measurement range caused by insufficient wavelength bands of the light sources (20.n, 21.m) are bridged.

5. Device according to claim 1, **characterized in that** the individually controllable light sources (20.n, 21.m) are controlled in any desired, but predefined, sequence.

6. Device according to one of the preceding claims, **characterized in that** the individually controllable broadband light sources (20.n, 21.m) are controlled with the same frequency of a nearly temporally symmetrical light-dark control signal, wherein the control signals of the light sources (20.n, 21.m) are phase-shifted relative to one another.

7. Device according to one of the preceding claims, **characterized in that** the electromechanically adjustable lens (45) is a liquid lens which is correlated with the light sources (20.n, 21.m) by the control unit.

8. Device according to one of the preceding claims, **characterized in that** the control device (70) and the image recording unit (50, 50a) are in connection with one another via a synchronisation line (74) in order to synchronise the individually controllable broadband light sources (20.n, 21.m) with the scanning frequency of the image recording unit.

9. Device according to one of claims 1 to 8, **characterized in that** the image recording unit includes a recording aperture raster diaphragm (54) with aperture diaphragms (56), which are arranged optically equivalent to the light sources (34) and which are redirected to any arrangement of detector pixels (58) through an array of optical fibres.

10. Device according to one of the preceding claims, **characterized in that** the image recording unit includes a recording aperture raster diaphragm (54) with aperture diaphragms (56) arranged optically equivalent to the light sources (34), which is imaged onto an image recording sensor (52) by a laterally movable microlens array (80), wherein the laterally movable microlens array (80) is set into a periodic movement (88) in at least one direction by a drive (82), wherein the control frequency (Sn, Sm) of the light sources (20.n, 21.m) is an integral multiple of the control frequency (SML) of the microlens array, and the control signals of the light sources (Sn, Sm) are correlated with the control signal (SML) of the microlens array by the control unit (70).

11. Device according to one of claims 1 to 10, **characterized in that** the image recording unit (50, 50a) comprises a fast lock-in camera sensor (pOCT sensor), in which each pixel (58) outputs an amplitude signal (A) and a phase signal (ϕ) of the incoming photon stream obtained directly with a lock-in evaluation circuit in the pixel 58.

12. Device according to one of claims 1 to 11, **characterized in that** the image recording unit (50, 50a) comprises a time-of-flight camera sensor (TOF or DAI sensor), in which each pixel (58) outputs a plurality of amplitude signals from which the phase signal of the incoming photon stream can be calculated by the control unit (70).

13. Device according to one of claims 1 to 9, **characterized in that** the image recording unit (50, 50a, 50b) comprises a CMOS camera sensor, in which the phase signal of the measurement points is calculated by the control unit by evaluating the amplitude image obtained by fast, exclusive read-out of the pixels exposed by the measurement points 56.

14. Device according to one of claims 1 to 8 and 10, **characterized in that** the image recording unit (50b) comprises a CMOS camera sensor, in which the phase signal of the measurement points is calculated by the control unit by evaluating the amplitude image (89) obtained by a lateral movement (88) of the microlens array (80).

15. Device according to one of claims 1 to 9, **characterized in that** the image recording unit (50, 50a, 50b) comprises a pulse-detecting radiation image sensor (timepix detector), in which the arrival time of a pulse is measured in each pixel, wherein for this purpose short, intensive control signals (Sm , Sm) are used which preferably generate a sinusoidal course of the light flashes, and which are also preferably shifted in phase to the nearest neighbour by 2/3π, and the depth information is calculated by the control unit from the arrival times of the light flashes.

## Revendications

1. Dispositif (10) pour la détection tridimensionnelle d'une structure de surface (11 ; 11' ; 11" ; 11'''), en particulier pour la détection de dents (13), comportant
a) une optique confocale chromatique (40, 42, 44) qui comprend un axe optique (41) et
b) une source lumineuse (16 ; 16a ; 16b ; 16c) qui présente une grille de sources lumineuses individuelles (34) espacées latéralement par rapport à l'axe optique (41),
c) dans lequel l'optique (40, 42, 44) forme l'image des sources lumineuses individuelles (34) sur la structure de surface (11 ; 11' ; 11" ; 11''') de telle manière qu'une grille de points de mesure (49) qui sont espacés latéralement les uns des autres y est produite,
d) dans lequel une unité d'acquisition d'images (50 ; 50a ; 50b) est disposée optiquement par rapport les sources lumineuses individuelles (34) de manière à détecter la grille de points de mesure (49),
dans lequel
e) un moyen de commande et d'évaluation (70) est prévu, qui est conçu
- pour commander la source lumineuse (16 ; 16a ; 16b ; 16c) de telle sorte que les sources lumineuses individuelles (34) émettent une lumière de composition spectrale différente selon une séquence chronologique et,
- à l'aide de l'unité d'acquisition d'images (50 ; 50a ; 50b), pour détecter la grille de points de mesure (49) de façon corrélée avec la séquence chronologique de la lumière émise par les sources lumineuses individuelles (34),
**caractérisé en ce que**
la source lumineuse (16b, 16c) présente au moins deux sources lumineuses à large bande activables individuellement (21.m) devant un élément de grille (30, 32) pour produire les sources lumineuses individuelles (34),
qui sont suffisamment petites pour que l'image de chaque source lumineuse individuelle soit pratiquement entièrement formée sur le même point de mesure, et
dont le spectre de longueurs d'onde présente respectivement une répartition d'intensité sinusoïdale ou triangulaire qui se chevauchent au moins partiellement, de telle sorte que, par une activation sinusoïdale ou triangulaire périodique déphasée des sources lumineuses activables (21.m), il se produit un décalage périodique du spectre de la lumière des sources lumineuses individuelles (34) par superposition de la lumière des sources lumineuses à large bande activables individuellement (21.m).

2. Dispositif selon l'une des revendications précédentes, **caractérisé en ce que** l'optique chromatique (40) contient des composants polarisants (42, 43, 47, 53), intégrés de telle sorte que seule la lumière diffusée par la surface 11 et provenant de la source lumineuse (16, 16a, 16b) atteint sensiblement l'unité d'acquisition d'images (50, 50a, 50b).

3. Dispositif selon l'une des revendications précédentes, **caractérisé en ce que** le groupe de lentilles chromatiques (44) est un groupe de lentilles hyperchromatiques.

4. Dispositif selon l'une des revendications précédentes, **caractérisé en ce que** le groupe de lentilles chromatiques (44) contient une lentille à réglage électromécanique (45), conçue pour combler des lacunes dans la plage de mesure de profondeur qui sont causées par des bandes de longueur d'onde insuffisantes des sources lumineuses (20.n, 21.m).

5. Dispositif selon la revendication 1, **caractérisé en ce que** les sources lumineuses activables individuellement (20.n, 21.m) sont activées dans un ordre quelconque mais prédéfini de manière fixe.

6. Dispositif selon l'une des revendications précédentes, **caractérisé en ce que** les sources lumineuses à large bande activables individuellement (20.n, 21.m) sont activées à la même fréquence d'un signal de commande lumière-obscurité pratiquement symétrique dans le temps, les signaux de commande des sources lumineuses (20.n, 21.m) étant déphasés les uns par rapport aux autres.

7. Dispositif selon l'une des revendications précédentes, **caractérisé en ce que** la lentille à réglage électromécanique (45) est une lentille liquide qui est corrélée avec des sources lumineuses (20.n, 21.m) par l'unité de commande.

8. Dispositif selon l'une des revendications précédentes, **caractérisé en ce que** le moyen de commande (70) et l'unité d'acquisition d'images (50, 50a) sont reliés par une ligne de synchronisation (74) afin de synchroniser les sources lumineuses à large bande activables individuellement (20.n, 21.m) avec la fréquence de balayage de l'unité d'acquisition d'images.

9. Dispositif selon l'une des revendications 1 à 8, **caractérisé en ce que** l'unité d'acquisition d'images comprend un diaphragme perforé d'acquisition (54) avec des sténopés (56) lesquels sont disposés de manière optiquement équivalente aux sources lumineuses (34) et lesquels sont redirigés par un réseau de fibres optiques vers une disposition quelconque de pixels détecteurs (58).

10. Dispositif selon l'une des revendications précédentes, **caractérisé en ce que** l'unité d'acquisition d'images contient un diaphragme perforé d'acquisition (54) avec des sténopés (56) disposés de manière optiquement équivalente aux sources lumineuses (34), dont l'image est formée par un réseau de microlentilles mobile latéralement (80) sur un capteur d'acquisition d'images (52) quelconque, le réseau de microlentilles mobile latéralement (80) étant mis dans un mouvement périodique (88), au moins dans une direction, par un entraînement (82), la fréquence d'activation (Sn, Sm) des sources lumineuses (20.n, 21.m) étant un multiple entier de la fréquence d'activation (S_{ML}) du réseau de microlentilles, et les signaux d'activation des sources lumineuses (Sn, Sm) étant corrélés avec le signal d'activation (S_{ML}) du réseau de microlentilles par l'unité de commande (70).

11. Dispositif selon l'une des revendications 1 à 10, **caractérisé en ce que** l'unité d'acquisition d'images (50, 50a) comprend un capteur de caméra à verrouillage rapide (en anglais : Fast Lock-In caméra) (capteur pOCT) dans lequel chaque pixel (58) émet un signal d'amplitude (A) et un signal de phase (ϕ) du courant photonique entrant qui est obtenu directement avec un circuit d'évaluation du type lock-in dans le pixel (58).

12. Dispositif selon l'une des revendications 1 à 11, **caractérisé en ce que** l'unité d'acquisition d'images (50, 50a) comprend un capteur de caméra temps de vol (en anglais : Time-Of-Flight caméra) (capteur TOF ou DAI) dans lequel chaque pixel (58) émet plusieurs signaux d'amplitude à partir desquels le signal de phase du courant photonique entrant peut être calculé par l'unité de commande (70).

13. Dispositif selon l'une des revendications 1 à 9, **caractérisé en ce que** l'unité d'acquisition d'images (50, 50a, 50b) comprend un capteur de caméra CMOS dans lequel le signal de phase des points de mesure est calculé par l'unité de commande en évaluant l'image d'amplitude qui a été obtenue par lecture rapide et exclusive des pixels éclairés par les points de mesure 56.

14. Dispositif selon l'une des revendications 1 à 8 et 10, **caractérisé en ce que** l'unité d'acquisition d'images (50b) comprend un capteur de caméra CMOS dans lequel le signal de phase des points de mesure est calculé par l'unité de commande en évaluant l'image d'amplitude (89) qui a été obtenue par un mouvement latéral (88) du réseau de microlentilles (80).

15. Dispositif selon l'une des revendications 1 à 9, **caractérisé en ce que** l'unité d'acquisition d'images (50, 50a, 50b) comprend un capteur d'image de rayonnement qui détecte des impulsions (détecteur Timepix), dans lequel le temps d'arrivée d'une impulsion est mesuré dans chaque pixel, de courts et intenses signaux de commande (Sₘ, Sₘ) qui produisent de préférence un tracé sinusoïdal des éclairs lumineux et qui sont en outre de préférence déphasés de 2/3π par rapport au voisin le plus proche étant utilisés à cet effet, et l'information de profondeur étant calculée par l'unité de commande à partir des temps d'arrivée des éclairs lumineux.
